# EUROPEAN PATENT APPLICATION

(11) **EP 4 512 906 A1**
(43) Date of publication of application: **26.02.2025**
(21) Application number: 23792168.9
(22) Date of filing: 19.04.2023
(51) Int. Cl.: C12Q 1/6809, C12Q 1/686, C12N 15/10, G16B 30/00

(54) **METHOD FOR VECTOR INSERTION SITE DETECTION AND CLONAL QUANTIFICATION USING TAGMENTATION**

(30) Priority: 19.04.2022 KR 20220047914
(71) Applicant: Seoul National University R & DB Foundation, Seoul 08826 (KR); Seoul National University Hospital, Seoul 03080 (KR)
(72) Inventor: KIM, Jong-il, Seoul 04105 (KR); KIM, Jaeryuk, Seoul 02831 (KR); KANG, Hyoung Jin, Seoul 05510 (KR); PARK, Miyoung, Seoul 08291 (KR)
(74) Representative: Manitz Finsterwald Patent- und Rechtsanwaltspartnerschaft mbB
(86) International application number: PCT/KR2023/005296
(87) International publication number: WO 2023/204594

(57) **Abstract**

The present disclosure relates to a method of detecting an integration site of a vector in a genome. According to the method of the present disclosure, it is possible to simply and quickly analyze a quantitative integration site of a viral vector with respect to a plurality of DNA motifs (sites) in a genome. Therefore, it can be useful for monitoring the safety and effectiveness of a gene therapy agent.

## Description

### TECHNICAL FIELD

The present disclosure relates to a method of detecting an integration site of an integrative vector in a genome and quantifying clones.

### BACKGROUND

Gene therapy can be defined as "a technique that aims to treat a disease by introducing a foreign gene to 1) correct and restore a defective gene to its original (normal) state or 2) provide a new function to cells". In practice, it refers to all techniques that involve introducing genes or gene-transduced cells into a human body to treat diseases or develop treatment models.

Plasmids or viral vectors are used to introduce genes into cells. In particular, as for immuno-oncology therapeutic agents, such as chimeric antigen receptor (CAR)-T cell therapy, the introduced genes need to be permanently expressed even after T cells divide. Therefore, retroviral vectors, such as gamma-retroviruses or lentiviruses, are commonly used to insert the introduced genes into chromosomes.

Unlike gamma-retroviral vectors, lentiviral vectors can be inserted into non-dividing cells. Recently, replication-incompetent vectors have been developed and are widely used for producing gene therapy agents, such as CAR-T cells. However, due to the nature of viral vectors that are randomly inserted into random sites in a chromosome, there is a concern that the vectors may be inserted into or around functional genes, especially oncogenes, and may potentially cause oncogenesis. To date, there have been no reports of tumor originating from gene-transduced cells using replication-incompetent lentiviruses, but because of such a concern, it is recommended to verify an integration site of a viral vector in a genome before a therapeutic agent is injected to a patient.

According to the European Medicines Agency (EMA) guideline "Guideline on Development and Manufacture of Lentiviral Vectors (CHMP/BWP/2458/03)", when therapeutic agents are produced using lentiviral vectors, it is recommended to analyze oncogenesis caused by proviral insertion. The guideline also suggests using nucleic acid amplification test (NAT) methods to identify integration sites of proviral vectors using appropriate cell lines.

According to "Guideline on Nonclinical Assessment of Gene Therapy Products" published by the National Institute of Food and Drug Safety Evaluation in 2021, it is stated that as for therapeutic agents using viral vectors, genotoxicity must be evaluated by identifying genomic integration sites and evaluating possible cross-talk between the transgene and neighboring sequences. Specifically, as for CAR-T cells, the guideline suggests that to assess oncogenesis, analysis of genomic integration sites of viruses and a test for abnormal cell proliferation should be considered.

Gene therapy agents need to be regularly monitored for oncogenesis before and after injection. According to "Guideline on Clinical Trial of Gene Therapy Products-Patient Follow-up for Delayed Adverse Reactions" published by the National Institute of Food and Drug Safety Evaluation in 2016, as for therapeutic agents using viral vectors with potential for integration or latent reactivation, it is recommended to perform analysis for evaluation of safety related to vector tracking and vector persistence after treatment.

Thus, as for gene therapy agents produced using viral vectors integrated into chromosomes, it is essential to identify integration sites in a genome through integration site analysis. In particular, after treatment, it is important to measure quantitative changes, such as the clonal size associated with the integration site. Recently, in addition to the viral vectors, non-viral vector systems, such as piggyBac transposon and Sleeping Beauty transposon, which can also be integrated into chromosomes, have gained attention. Thus, the demand for integration site analysis of these systems has also increased. However, no standardized method for this analysis exists globally.

Although no gold standard method is currently recognized, the most widely used methods include LAM (linear amplification mediated)-PCR, nrLAM (non-restrictive enzyme linear amplification mediated)-PCR, and LM (ligation mediated)-PCR. Recently, these methods have been combined with next-generation sequencing (NGS). Thus, it is possible to analyze many integration sites with higher sensitivity in just one test.

These methods require several stages, such as fragmentation using restriction enzymes or ultrasound and attachment of linkers, which makes the process complicated. Also, these methods also take at least two days to prepare a library for NGS. For clinical applications involving a plurality of samples, the process needs to be simple and fast. However, due to these limitations, the above-described methods are used only selectively. Also, sample loss occurs during the several stages, and, thus, a relatively large initial amount of DNA (1 to 3 µg or more) is needed. Further, the use of ultrasound necessitates expensive equipment.

With a rapid increase in clinical application of gene therapy agents, such as CAR-T cells, the demand for techniques to analyze integration sites of vectors quickly and easily on a large scale is growing. In particular, as for gene therapy, various tests need to be performed on limited blood samples, and, thus, there is a need for a method to perform integration site analysis with a small amount of DNA.

### DISCLOSURE OF THE INVENTION

### PROBLEMS TO BE SOLVED BY THE INVENTION

The present inventors, during research on a method for analyzing a quantitative integration site of a vector in a genome, have found that a quantitative integration site of a viral vector in a genome can be analyzed more simply and quickly than conventional methods by performing tagmentation using a bead-linked transposome and optimizing PCR conditions, and thus have completed the present disclosure.

Accordingly, the present disclosure is conceived to provide a method for detecting an integration site of a vector in a genome.

Also, the present disclosure is conceived to provide a method for quantifying clones in which vectors are integrated into genomes.

### MEANS FOR SOLVING THE PROBLEMS

The present inventors, during research on a method for analyzing a quantitative integration site of a vector in a genome, have found that a quantitative integration site of a viral vector in a genome can be analyzed more simply and quickly than conventional methods by performing tagmentation using a bead-linked transposome and optimizing PCR conditions.

Therefore, the present disclosure relates to a method for detecting an integration site of a vector in a genome and quantifying clones.

### EFFECTS OF THE INVENTION

The present disclosure relates to a method of analyzing an integration site of a vector in a genome. The features and advantages of the present disclosure are summarized as follows.
(a) According to the method of the present disclosure, it is possible to simply and quickly analyze a quantitative integration site of a vector in a genome.
(b) According to the method of the present disclosure, it is possible to analyze a quantitative integration site of a vector with respect to a plurality of DNA motifs (sites).
(c) The present disclosure can be useful for monitoring the safety and effectiveness of a gene therapy agent, such as CAR-T.

### BRIEF DESCRIPTION OF THE DRAWINGS

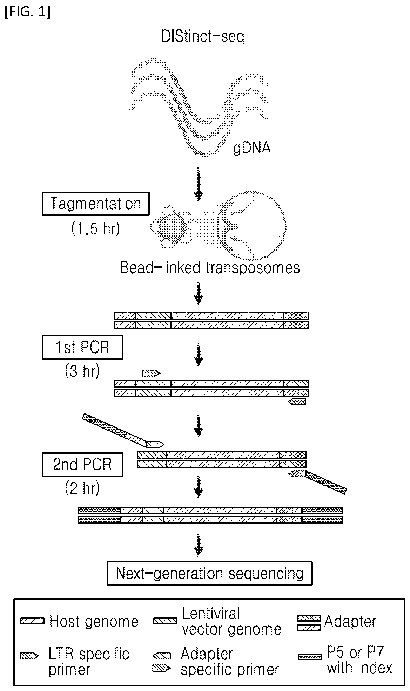

**FIG. 1** is a schematic diagram showing a method (DIStinct-seq) for detecting an integration site of a vector in a genome and quantifying clones using tagmentation according to the present disclosure.
**FIG. 2** is a schematic diagram showing a bioinformatics pipeline in the method (DIStinct-seq) for detecting an integration site of a vector in a genome and quantifying clones using tagmentation according to the present disclosure.

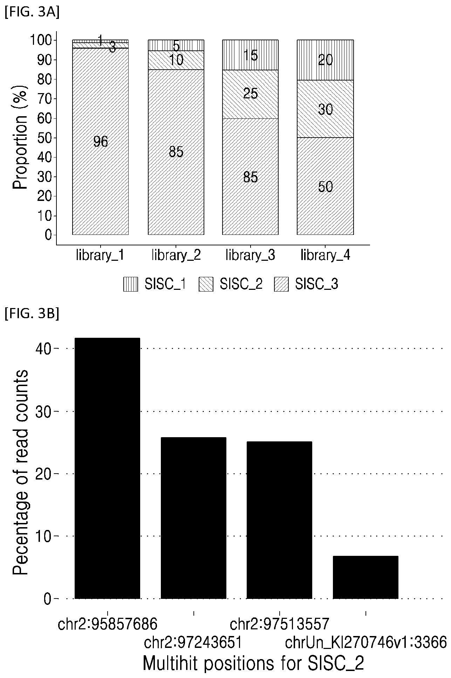

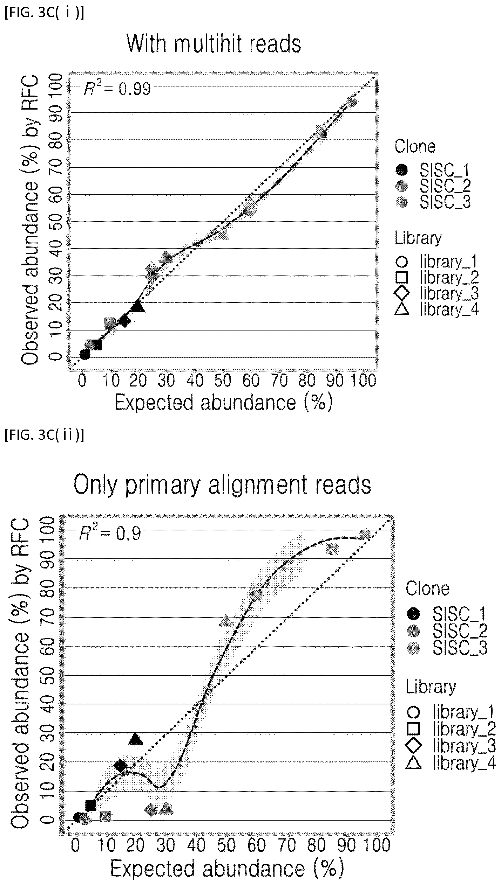

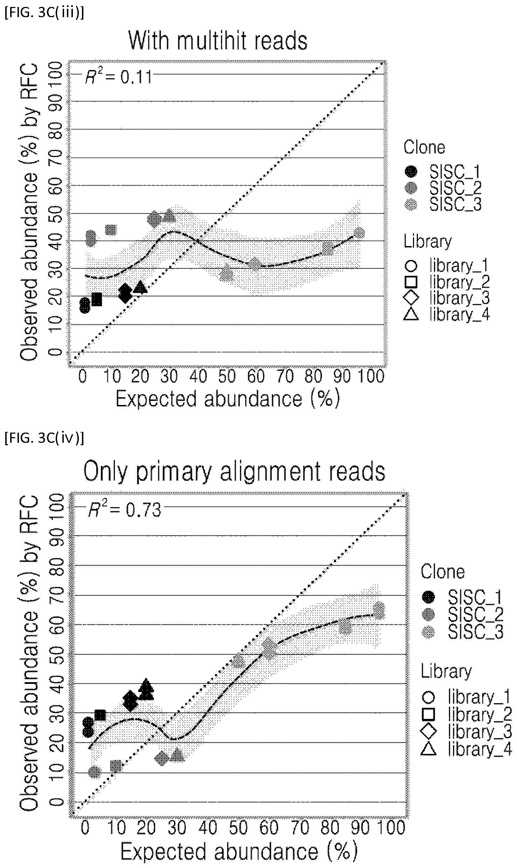

**FIG. 3A to FIG. 3C(iv)** verify the quantitative integration site analysis capability of an analysis method according to an example of the present disclosure, **FIG. 3A** shows the proportion of clones used in a test, **FIG. 3B** shows the percentage of read counts depending on multiple-alignment sites caused by the mapping ambiguity of one of the clones, and **FIG. 3C( i )** to **FIG. 3C(iv)** show the result of checking the expected sizes of clones for each of an unprocessed fragment and a fragment from which PCR duplicates were removed when multiple-alignment fragments are integrated and when only primary alignment reads are used.
**FIG. 4A** to **FIG. 4E** show the result of analyzing DNA motifs around integration sites in CAR-T cells, which were produced using lentiviral vectors according to an example of the present disclosure, by the analysis method of the present disclosure.
**FIG. 5A** to **FIG. 5H** show the result of analyzing the chromosome type and integration ratio in functional genomic regions in CAR-T cells, which were produced using lentiviral vectors according to an example of the present disclosure, by the analysis method of the present disclosure.
**FIG. 6A( i )** to **FIG. 6A(iii)** and **FIG. 6B( i )** to **FIG. 6B(vi)** show the result of analyzing a relationship between clone abundance and integration ratio in functional genomic regions in CAR-T cells, which were produced using lentiviral vectors according to an example of the present disclosure, by the analysis method of the present disclosure **(FIG. 6A( i** ) to **FIG. 6A(iii):** Classification by clone abundance, **FIG. 6B( i )** to **FIG. 6B(vi):** Analysis of integration site ratio in functionally important genomic regions depending on clone abundance).
**FIG. 7** shows the result of pathway enrichment analysis of genes at integration sites depending on the clone abundance in CAR-T cells, which were produced using lentiviral vectors according to an example of the present disclosure, by the analysis method of the present disclosure.
**FIG. 8A** to **FIG. 8D(vi)** show the result of analyzing integration sites over time in CAR-T cells, which were produced using lentiviral vectors according to an example of the present disclosure, in vivo by the analysis method of the present disclosure **(****FIG. 8A****:** Overview of in vivo test, **FIG. 8B****:** Quantitative changes in cells into which CAR-T vectors were integrated in vivo, **FIG. 8C****:** Shannon entropy index over time, **FIG. 8D( i** ) to **FIG. 8D(vi):** Proportion of clones in top 1 percent by clone abundance in all of clones over time).
**FIG. 9A( i** ) to **FIG. 9A(iii)** and **FIG. 9B( i** ) to **FIG. 9B(vi)** show the result of quantitatively analyzing integration sites over time in CAR-T cells, which were produced using lentiviral vectors according to an example of the present disclosure, in vivo by the analysis method of the present disclosure **(FIG. 9A( i** ) to **FIG. 9A(iii):** Classification by clone abundance, **FIG. 9B( i** ) to **FIG. 9B(vi):** Analysis of integration site ratio in functionally important genomic regions depending on clone abundance).

### BEST MODE FOR CARRYING OUT THE INVENTION

Hereafter, the present disclosure will be described in more detail.

An aspect of the present disclosure relates to a method of detecting an integration site of a vector in a genome, including the following processes:
a process of tagmentation using a bead-linked transposome;
a process of preparing a library through gene amplification;
a process of library pooling and sequencing; and
a process of determining an integration site in a genome through bioinformatics analysis.

In the present disclosure, the vector may be a viral vector, and the virus may be a lentivirus and/or a retrovirus, but may not be limited thereto.

According to an embodiment of the present disclosure, the method enables quantitative analysis of the integration site.

Hereafter, the method for detecting an integration site of a vector in a genome according to the present disclosure will be described in detail.

### Tagmentation

In the present process, fragmentation of nucleic acid and adapter tagging are performed simultaneously. In the present process, a nucleic acid extracted from a sample is broken down into an appropriate size for analysis and at the same time, an adapter for binding primers for library preparation is attached.

Throughout the whole document, the term "fragmentation" refers to breaking down a nucleic acid into an appropriate size for analysis, and may include random breaking down by physical or enzymatic methods.

The physical method typically uses energy generated by ultrasound produced by equipment to fragment a nucleic acid, with the fragmentation length adjusted by controlling the generated energy and exposure time. Currently, equipment from Covaris, Diagenode, and Qsonica is widely used. The enzymatic method involves obtaining nucleic acid fragments of a desired size by treating enzymes, such as nuclease, fragmentase, and transposase, which randomly break down a nucleic acid under appropriate conditions.

Throughout the whole document, the term "adapter" refers to a chemically synthesized short single-stranded or double-stranded oligonucleotide that can be ligated to the end of a DNA or RNA molecule, and the adapter includes a platform-specific sequence for fragment recognition by a next-generation sequencer.

In the present process of tagmentation, a part of the adapter sequence is tagged, and the remaining adapter sequence is attached via polymerase chain reaction.

According to an embodiment of the present disclosure, the present process may be performed by a bead-linked transposome (BLT).

The BLT is a structure in which a transposome, which is a complex of an enzyme that breaks down a nucleic acid, such as Tn5 transposase, and an adapter, is attached to a bead.

When the enzyme that breaks down a nucleic acid is present in solution, there is a need to adjust the ratio of the amount of DNA to the amount of reagent in order for the DNA to be fragmented to an appropriate size. This is not only time consuming, but also limits the maximum amount of DNA per sample (^{~}50 ng), which makes it difficult to obtain sufficient samples for quantitative integration site analysis.

Meanwhile, the BLT is used according to the present disclosure, and, thus, tagmentation of a predetermined amount of DNA, i.e., DNA fragmentation and adapter tagging, occurs on a bead with a transposome directly attached to the bead. Therefore, it is possible to prepare libraries with consistent fragment sizes and yields. Also, it is possible to save time needed to quantify the input DNA.

Further, the use of the BLT according to the present disclosure significantly increases the amount of DNA for integration site analysis (100 to 500 ng) compared to conventional methods. As a result, it is possible to identify more integration sites in a single reaction and also possible to improve the accuracy in quantitative analysis.

### Library Preparation

The present process involves performing gene amplification on the tagmented nucleic acid fragments. Through the present process, DNA (host/vector-fused DNA) into which a trace of vector present in the tagmented nucleic acid fragments is integrated is specifically amplified.

In the present disclosure, the gene may be a host/ vector-fused DNA fragment.

Specifically, the present process may be performed by the following processes:
a 1st round polymerase chain reaction (PCR); and
a 2nd round PCR.

The 1st PCR may be performed under, for example, the following conditions, but may not be limited thereto:
[98°C, 5 minutes] 1 cycle; [98°C, 10 seconds], [60°C, 15 seconds], [68°C, 2 minutes] 30 cycle; [68°C, 5 minutes] 1 cycle; [4°C, hold] 1 cycle.

The 1st PCR may use a forward primer consisting of a base sequence of SEQ ID NO: 1 and a reverse primer consisting of a base sequence of SEQ ID NO: 2, but may not be limited thereto.

Throughout the whole document, the term "primer" refers to a short, nucleic acid strand having a free 3' hydroxyl group, which forms a base pair with a complementary template so as to serve as a starting point for replicating a template strand.

Also, the 2nd PCR may be performed by a nested-PCR, and may be performed under, for example, the following conditions, but may not be limited thereto:
[98°C, 5 minutes] 1 cycle; [98°C, 10 seconds], [60°C, 15 seconds], [68°C, 2 minutes] 15 cycle; [68°C, 5 minutes] 1 cycle; [4°C, hold] cycle.

The 2nd PCR may use a forward primer consisting of a base sequence of SEQ ID NO: 3 and a reverse primer consisting of a base sequence of SEQ ID NO: 4, but may not be limited thereto.

In the method of the present disclosure, to minimize the generation of non-specific amplification products that may occur during the 1st PCR, the 2nd PCR was performed using the DNA amplified once as a template (nested-PCR). Also, to minimize the generation of recombinant DNA products during the PCR, the number of cycles in the 1st PCR was reduced (from 40 cycles to 30 cycles) and the elongation time of the first and 2nd PCRs was increased (from 1 minute to 2 minutes).

The sequence of each primer in the present disclosure can be appropriately selected depending on the type of vector used.

Specifically, for example, the forward primer consisting of a base sequence of SEQ ID NO: 1 and used for the 1st PCR in the present disclosure is "a 20 bp sequence complementary to a 3' long terminal repeat (LTR) at the end of an integrated lentiviral sequence". If the type of integrated virus differs, the base sequence of SEQ ID NO: 1 can be adjusted to match with the sequence of the corresponding virus.

Also, for example, the forward primer consisting of a base sequence of SEQ ID NO: 3 and used for the 2nd PCR in the present disclosure is "a 20 bp sequence complementary to a 3' LTR at the end of an integrated lentiviral sequence, located on a more downstream side than the forward primer for the 1st PCR, and excluding a 13 bp sequence at the 5' end of the 3' LTR". If the type of integrated virus differs, the base sequence of SEQ ID NO: 3 can be adjusted to match with the sequence of the corresponding virus.

### Library Pooling and Sequencing

The present process involves pooling the same amount of DNA from individual samples into one pool for sequencing of the prepared library. Through the present process, raw sequencing reads required for bioinformatics analysis to be performed in the subsequent process of determining an integration site in a genome are obtained.

In the present disclosure, the term "sequencing" refers to a process of obtaining DNA sequence information in a next-generation sequencer. During the process of preparing a library, the attached adapter region binds to a complementary primer on the sequencer, which enables large-scale replication. Sequencing reads are obtained by observing an order in which bases are synthesized on the aligned DNA. This process can be performed by an appropriate system (*e.g.,* NovaSeq 6000).

### Determination of Integration Site in Genome

The present process involves determining a integration site of a vector in a genome through a series of bioinformatics pipelines for the pooled sequences.

Throughout the whole document, the term "bioinformatics" is an applied science that uses computers to analyze and process large-scale biological data to obtain useful information, and may include all fields of biology research using computers.

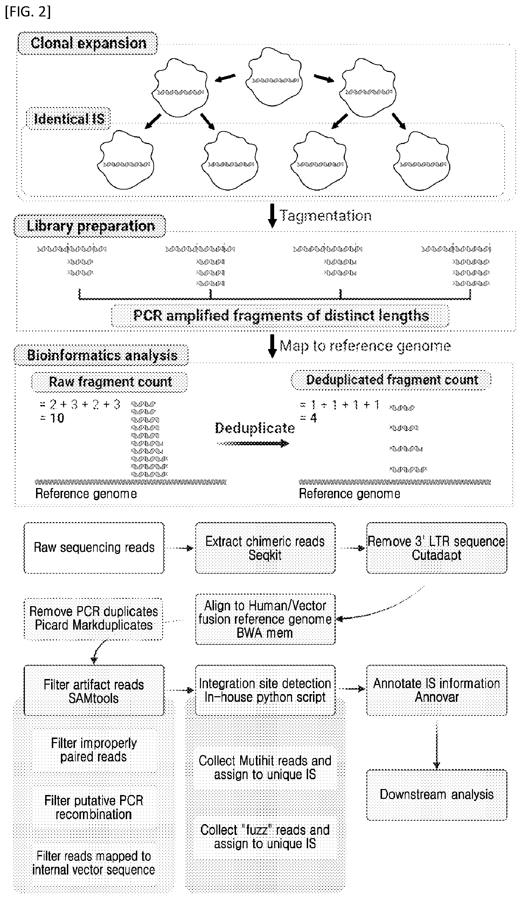

The bioinformatics pipelines for the present process are shown in **FIG. 2****,** and the present process may be performed by the following processes:
a process of extracting a chimeric read;
a process of deleting a 3' LTR-specific sequence;
a process of creating a host/vector-fused genome;
a process of aligning the read on the host/vector-fused genome;
a process of removing a PCR duplicate;
a process of filtering the read; and
a process of determining the integration site.

Each of the above-described processes may be performed by a tool, such as Seqkit, Cutadapt, BWA, Picard, Samtools, and/or In-house Python script, but may not be limited thereto.

According to an embodiment of the present disclosure, Seqkit (version 0.14.0) (Shen, W., Le, S., Li, Y., and Hu, F.Q. (2016). SeqKit: A Cross-Platform and Ultrafast Toolkit for FASTA/Q File Manipulation. PLoS One 11, e0163962. https://doi.org/10.1371/journal.pone.0163962.) may be used to extract a chimeric read including a vector-fused genome from raw sequencing reads.

According to another embodiment of the present disclosure, Cutadapt (version 1.18) (Martin, M. (2011). Cutadapt removes adapter sequences from high-throughput sequencing reads. EMBnet. journal 17, 10-12. https://doi.org/10.14806/ej.17.1.200.) may be used to delete a 3' LTR-specific sequence from each read.

According to another embodiment of the present disclosure, BWA (version 0.7.17) (Li, H., and Durbin, R. (2009). Fast and accurate short read alignment with Burrows-Wheeler transform. Bioinformatics 25, 1754-1760. https://doi.org/10.1093/bioinformatics/btp324.) mem options may be used to create a host/vector-fused reference genome by binding a host reference genome to a vector sequence and align a read on the host/vector-fused reference genome.

According to yet another embodiment of the present disclosure, Picard (version 2.24.0) (Picard toolkit. (2019). Broad Institute, GitHub repository.) may be used to remove a PCR duplicate. However, it can be optionally omitted when the clone abundance is quantified based on the raw fragment count.

According to still another embodiment of the present disclosure, Samtools (version 1.3.1) (Li, H., Handsaker, B., Wysoker, A., Fennell, T., Ruan, J., Homer, N., Marth, G., Abecasis, G., Durbin, R., and Proc, G.P.D. (2009). The Sequence Alignment/Map format and SAMtools. Bioinformatics 25, 2078-2079. https://doi.org/10.1093/bioinformatics/btp352.) may be used to filter the read according to the following criteria to ensure analysis quality: mapping quality of 20 or greater, properly paired reads represented by SAM flag 0X2, paired reads with insert size exceeding 2000 bp, excluding reads aligned to the lentiviral vector genome and not primary alignment by SAM flag 0×100.

According to still another embodiment of the present disclosure, In-house Python script may be used to determine a unique integration site. For accurate quantitative analysis of the unique integration site, multihit reads caused by mapping ambiguity and fuzz reads of up to 3 bp that may be generated during the PCR and sequencing can be counted as unique reads.

### MODE FOR CARRYING OUT THE INVENTION

Hereafter, the present disclosure will be described in more detail with reference to examples. It will be obvious to a person with ordinary skill in the art that these examples are illustrative purposes only and are not to be construed to limit the scope of the present disclosure.

### Example. Establishment of a method for detecting an integration site of a vector in a genome and quantifying clones of the present disclosure (DIStinct-seq; Detection of integration sites in a time-efficient manner, quantifying clonal size using tagmentation sequencing) (see FIG. 2)

### 1. Tagmentation

A process of performing DNA fragmentation and adapter tagging by using a bead-linked transposome (BLT)

### 2. Library Preparation

A process of preparing a library through a 1st round PCR and a 2nd round PCR

### 3. Library Pooling and Sequencing

A process of pooling a library after quantification into one tube in a volume which ensures that each sample has the same amount of molecules and obtaining sequence information via sequencing

### 4. Bioinformatics Analysis

A process of determining an integration site of a viral vector in a genome through a series of processes

### Test Example 1. Verification of quantitative integration site analysis capability by analysis method of the present disclosure

To verify that it is possible to quantitatively measure the clone abundance with integrated viral vectors according to the present disclosure, DNAs from single-cell derived clones with known integration sites were mixed at specific ratios, and the integration sites were identified by the method of the present disclosure.

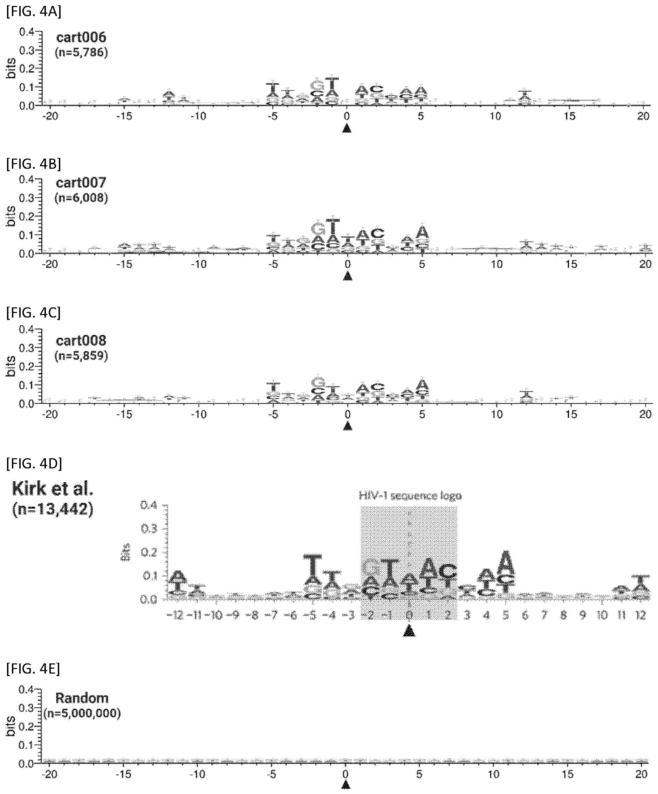

Specifically, a lentiviral vector expressing EmGFP (Addgene #113884) at an MOI of 0.4 was transduced into the HEK293FT cell line (Thermofisher Scientific), and only cells with integrated vectors were isolated through a Fluorescence Activated Cell Sorter (FACS) (BD FACSAria III Cell Sorter). The isolated cells were serially diluted and distributed into 96-well plates, followed by incubation to isolate colonies derived from single cells. Three (SISC_1, SISC_2, SISC_3) of these single-cell derived colonies (single integration site clones (SISCs)) were subjected to whole-genome sequencing (WGS) (30X) to identify their integration sites **(****FIG. 4A** to **FIG. 4E****),** and the DNAs were combined at specific ratios (library_1 to library_4) (Table 1).

**[Table 1]**

| | | DNA | | |
|---|---|---|---|---|
| | | SISC_1 | SISC_2 | SISC_3 |
| Library | library_1 | 1% | 3% | 96% |
| | library_2 | 5% | 10% | 85% |
| | library_3 | 15% | 25% | 60% |
| | library_4 | 20% | 30% | 50% |

DIStinct-seq was repeated twice on each DNA from the library_1 to the library_4 to prepare a total of 8 libraries. This is performed as follows:

### A. Tagmentation

Tagmentation in which adapter tagging and fragmentation occur simultaneously was performed by using an Illumina DNA prep kit from Illumina, Inc.

Specifically, 5 µl (500 ng) of DNA dilution was placed in a PCR tube, and then 25 µl of nuclease-free water was added to prepare a DNA sample tube with a volume of 30 µl. Thereafter, 11 µl each of bead-linked transposome (BLT) and tagmentation buffer 1 (TB1), which were separately taken out at room temperature, were mixed to prepare a tagmentation master mix, which was then vortexed and completely resuspended.

After 20 µl of the tagmentation master mix was transferred to the DNA sample tube, each sample was resuspended by pipetting 10 times. The sample tube was placed in a thermal cycler and incubated at a lid temperature of 100°C, a reaction volume of 50 µl, a reaction time of 15 minutes, a reaction temperature of 55°C, and a stop temperature of 0°C.

### B. Post-tagmentation Cleanup

A tagmentation stop buffer (TSB) was taken out to room temperature and incubated at 37°C until all precipitates were dissolved. Then, 10 µl of the TSB was added to the tagmentation tube. Each sample was resuspended by gently pipetting 10 times and incubated in a thermal cycler at a lid temperature of 100°C, a reaction volume of 60 µl, a reaction time of 15 minutes, a reaction temperature of 37°C, and a stop temperature of 10°C.

The sample tube was placed on a magnetic stand for up to 3 minutes until a solution became clear. The supernatant was removed and discarded, the sample tube was separated from the magnetic stand, and 100 µl of a tagment wash buffer (TWB), which had been taken out at room temperature, was carefully added onto beads and resuspended by pipetting slowly. The sample tube was placed on the magnetic stand for up to 3 minutes until the solution became clear.

The supernatant was removed and discarded, and the above-described process (addition of the TWB and removal of the supernatant) was repeated two more times.

### C. 1st Round PCR

A PCR reaction solution was prepared with the composition shown in Table 2 below. PCR primers have information and sequences as shown in Table 3 below. A sample tube was placed in a thermal cycler, and PCR was performed at a lid temperature of 100°C, a reaction volume of 50 µl, and temperature and time conditions shown in Table 4 below.

**[Table 2]**

| Material | Volume |
|---|---|
| 5X PrimesSTAR GXL Buffer (Takara inc.) | 10 µl |
| dNTP Mixture (Takara inc.) | 4 µl |
| Primer 1 | 1 µl (10 pmol), 0.2 µM |
| Primer 2 | 1 µl (10 pmol), 0.2 µM |
| Template | Bead |
| PrimeSTAR GXL DNA Polymerase (Takara inc.) | 1 µl |
| Sterile distilled water | 33 µl |
| Total | 50 µl |

**[Table 3]**

| No. | Sequence (5'-3') | Sequence Information |
|---|---|---|
| Primer 1 (forward) | AGTAGTGTGTGCCCGTCTGT (SEQ ID NO: 1) | 20 bp complementary to a 3' long terminal repeat (LTR) at the end of the integrated lentiviral sequence |
| Primer 2 (reverse) | GTCTCGTGGGCTCGGAGATG (SEQ ID NO: 2) | 20 bp complementary to an adapter sequence |

**[Table 4]**

| Number of cycle | Temperature | Duration |
|---|---|---|
| 1 | 98 °C | 5 min |
| 30 | 98 °C | 10 sec |
| | 60 °C | 15 sec |
| | 68 °C | 2 min |
| 1 | 68 °C | 5 min |
| 1 | 4 °C | hold |

### D. 2nd Round PCR

To increase specificity from the product of the 1st round PCR, semi-nested PCR was performed.

Specifically, a PCR reaction solution was prepared with the composition shown in Table 5 below. PCR primers have information and sequences as shown in Table 6 below. A sample tube was placed in a thermal cycler, and PCR was performed at a lid temperature of 100°C, a reaction volume of 50 µl, and temperature and time conditions shown in Table 7 below.

**[Table 5]**

| Material | Volume |
|---|---|
| 5X PrimesSTAR GXL Buffer (Takara Inc.) | 10 µl |
| dNTP Mixture (Takara Inc.) | 4 µl |
| Primer 3 | 1 µl (10 pmol), 0.2uM |
| Primer 4(A/B/C) | 1 µl (10 pmol), 0.2uM |
| Template | 33 µl |
| PrimeSTAR GXL DNA Polymerase (Takara Inc.) | 1 µl |
| Total | 50 µl |

**[Table 6]**

| No. | Sequence (5'-3') | Sequence Information |
|---|---|---|
| Primer 3 (forward) | AATGATACGGCGACCACCGAGAT CTACACNNNNNNNNNNTCGTCG GCAGCGTCAGATGTGTATAAGAG ACAG GACCCTTTTAGTCAGTGTGG (SEQ ID NO: 3) | a 29 bp-long P5 sequence + a 10 bp-long index sequence (different for each sample, indicated by N) + 33 bp complementary to an adapter sequence + a 20 bp sequence complementary to a 3' LTR at the end of an integrated lentiviral sequence, located on a more downstream side than Primer 1 sequence, and excluding a 13 bp sequence at the 5' end of the 3' LTR |
| Primer 4 (reverse) | CAAGCAGAAGACGGCATACGAG ATNNNNNNNNNNGTCTCGTGG GCTCGG (SEQ ID NO: 4) | 15 bp complementary to an adapter sequence + a 10 bp-long index sequence (different for each sample, indicated by N) + a 24 bp-long P7 sequence |

**[Table 7]**

| Number of cycle | Temperature | Duration |
|---|---|---|
| 1 | 98 °C | 5 min |
| 15 | 98 °C | 10 sec |
| | 60 °C | 15 sec |
| | 68 °C | 2 min |
| 1 | 68 °C | 5 min |
| 1 | 4 °C | hold |

### E. Bead Purification & Size Selection

SPRlselect beads from Beckman Coulter Inc. were used to remove impurities, such as PCR dimers, from the library prepared through the above-described process and refine the library to the optimal size (200 bp to 500 bp).

The SPRlselect beads were vortexed, and 25 µl (0.5X) of the SPRIselect beads was added to a sample tube, mixed thoroughly by pipetting, and incubated at room temperature for 5 minutes. The sample tube was placed on a magnetic stand and then, 70.32 µl of the mixture was transferred to a new PCR tube.

The SPRlselect beads were vortexed, and 20 µl (0.9X) of the SPRIselect beads was added to a sample tube, mixed thoroughly by pipetting, and incubated at room temperature for 5 minutes. The sample tube was placed on a magnetic stand and then, 81 µl of the supernatant was removed, but the beads were left undisturbed.

While the sample tube was kept on the magnetic stand, 125 µl of 80% ethanol was added and the ethanol was removed after 30 seconds. The above-described process (addition and removal of ethanol) was repeated once and then the sample tube was lightly spun down.

The sample tube was placed on a magnetic stand and the ethanol was removed. After the sample tube was separated from the magnetic stand, 61 µl of an elution buffer was added and mixed thoroughly by pipetting. After incubation at room temperature for 2 minutes, the sample tube was placed on a magnetic stand, and when a solution becomes clear, 60 µl of the solution is transferred to a new tube.

### F. Pooling and Sequencing

After each library was quantified with a Broad Range Qubit, a result value was used to calculate the volume which ensures that each sample has the same amount of molecules, and the corresponding volume was taken and pooled into one tube. Sequence information was obtained by using NovaSeq6000 from Illumina Inc. (Theragen Bio).

### G. Bioinformatics Analysis

Finally, integration site analysis was performed through bioinformatics analysis.

Specifically, a chimeric read including a vector-fused genome was extracted from raw sequencing reads by using SeqKit (version 0.14.0). Then, a 3' LTR-specific sequence was removed from each read by using Cutadapt (version 1.18). BWA (version 0.7.17) mem options were used to create a host/vector-fused reference genome by binding a human reference genome (hg38) to a vector sequence and align a read on the host/vector-fused reference genome. A PCR duplicate was removed by using Picard (version 2.24.0). However, this process was optionally omitted when the clone abundance was quantified based on the raw fragment count. Thereafter, Samtools (version 1.3.1) was used to filter the read according to the following criteria to ensure analysis quality: mapping quality of 20 or greater, properly paired reads represented by SAM flag 0X2, paired reads with insert size exceeding 2000 bp, excluding reads aligned to the lentiviral vector genome and not primary alignment by SAM flag 0×100. Finally, for accurate quantitative analysis of a unique integration site, In-house Python script was used to count multihit reads caused by mapping ambiguity and fuzz reads of up to 3 bp that may be generated during the PCR and sequencing as reads at unique integration sites.

As a result of applying bioinformatics pipelines to the library prepared by the above-described method by mixing clones with known integration sites at certain ratios **(****FIG. 3A****),** a plurality of sites was detected in SISC_2 by the multihit reads caused by mapping ambiguity **(****FIG. 3B****).** Also, with In-house python script, both raw fragment count (RFC) and deduplicated fragment count (DFC) were obtained for a case where a plurality of sites was integrated and for primary alignment reads with the highest number of reads, and as can be seen from **FIG. 3C( i )** to **FIG. 3C(iv),** it was found out that when the plurality of sites was integrated and the raw fragment count was used, the PCR fragment count was proportional to the expected clone abundance, compared to a case where only primary alignment reads were used or PCR duplicates were removed.

Therefore, according to the integration site analysis method of the present disclosure, it was verified that the clone abundance can be measured quantitatively.

### Test Example 2. Confirmation of quantitative insertion site analysis in CAR-T cell produced using lentiviral vector

The integration site analysis method (DIStinct-seq) of the present disclosure was directly applied to CAR-T cells, a gene therapy agent, to analyze integration sites. The present inventors attempted to check safety by analyzing the clone abundance depending on integration sites.

White blood cells were collected from three healthy people, T cells were isolated (approved by the IRB of Seoul National University), and a total of three CAR-T cell lines (cart006, cart007 and cart008) were produced by transduction of lentivirus with a CAR vector.

Specifically, CD4+ and CD8+ T cells from healthy donors were incubated in TexMACS medium with IL-7 (12.5 ng/mL), IL-15 (12.5 ng/mL), and 3% human AB serum (Life Science Production, Bedford, UK), and T cells were activated with CD3/CD28 MACS^{®} GMP TransAct reagent (Miltenyi Biotec). On day 1 of incubation, the activated T cells were transduced with a lentiviral vector encoding a CAR gene. The lentiviral vector used herein was LTG1563, a CD19 CAR vector, developed and supplied by Lentigen, a subsidiary of Miltenyi Biotec (Gaithersburg, MD, United States). On day 3 of incubation, the medium was replaced, and on day 6, the incubate was transferred to TexMACS medium (serum-free) supplemented with 12.5 ng/mL of IL-7 and IL-1 and then incubated until harvest on day 12. This process was performed by an automated instrument, CliniMACS Prodigy (Miltenyi Biotec, Bergisch Gladbach, Germany).

DIStinct-seq was performed on the prepared CAR-T cell lines according to the method of Test Example 1.

### A. Analysis of DNA Motif around Integration Site

DNA motifs around the integration sites were analyzed by Weblogo based on FASTA file created with Bedtools.

**As** can be seen from **FIG. 4A** to **FIG. 4E****,** DNA motifs (cart006, cart007 and cart008 in **FIG. 4A** to **FIG. 4C****)** around the integration sites of the lentivirus determined by the integration site analysis method (DIStinct-seq) of the present disclosure were perfectly matched with DNA motifs (Kirt et al. in **FIG. 4D****)** (Nature microbiology, 2016, 2.2: 1-6. PMID: 27841853) around the conventionally known integration sites of the identical lentivirus.

### B. Analysis of Chromosome Type and Integration Ratio in Functional Genomic Region

Integration ratios of a lentiviral vector in chromosomes (1 to 22, X and Y) and functionally important genomic regions (transcription unit, exon, transcription start site +/- 5kb, transcription start site of oncogene +/- 50kb, CpG island +/- 5kb, Genomic safe harbor) were analyzed.

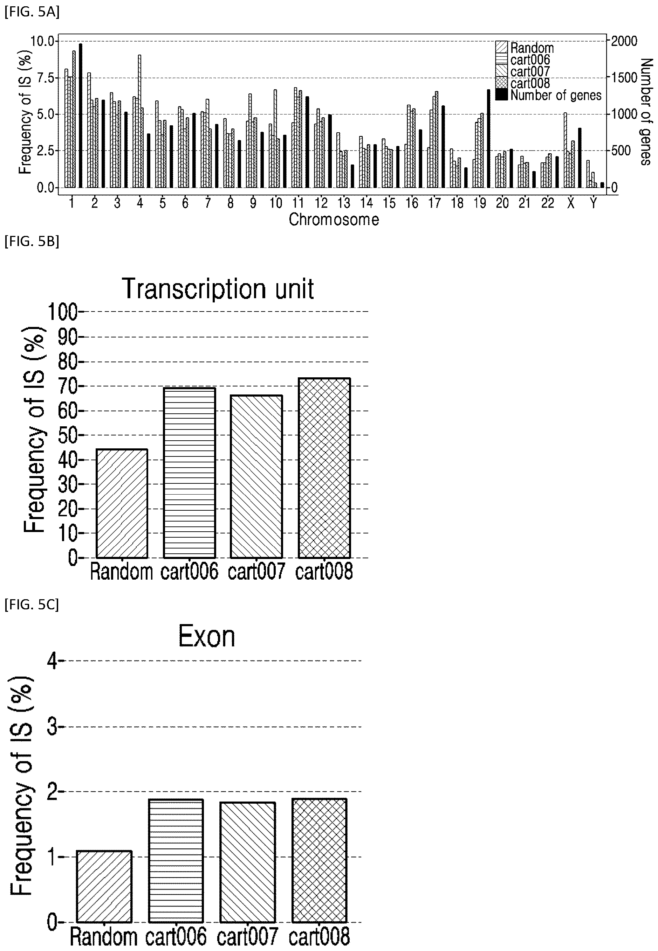

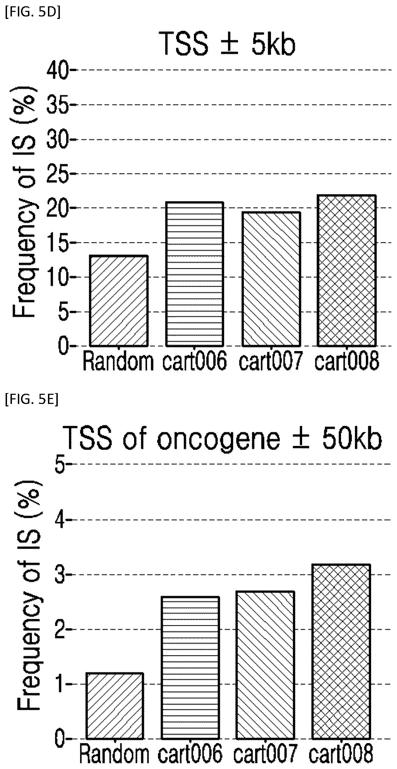

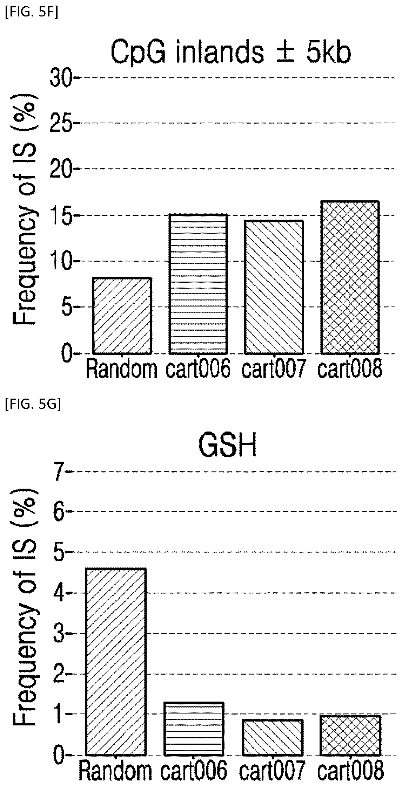

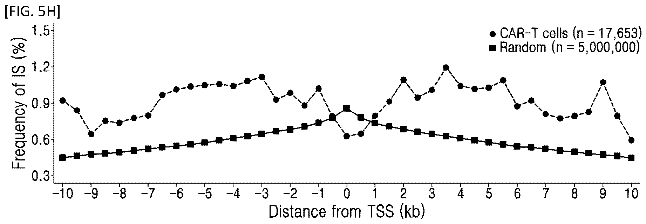

As can be seen from **FIG. 5A** to **FIG. 5H****,** the integration sites of the lentivirus determined by the integration site analysis method (DIStinct-seq) of the present disclosure were matched with the conventionally known integration sites of the identical lentivirus.

### C. Relationship between Clone Abundance and Integration Ratio in Functional Genomic Region

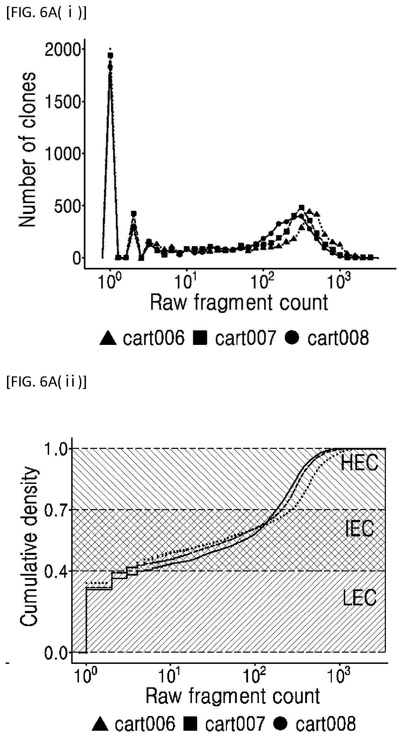

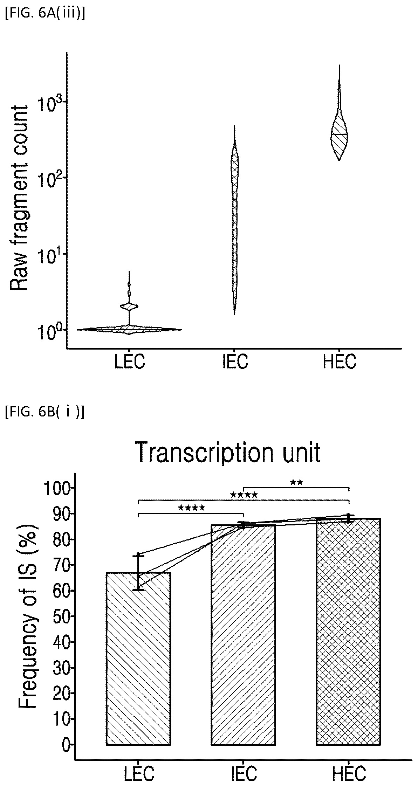

First, according to the results of the integration site analysis method (DIStinct-seq) of the present disclosure, the clones were classified into LEC (less expanded clone), IEC (intermediately expanded clone), and HEC (highly expanded clone) depending on the number of DNA fragments with the same integration sites (clone abundance can be estimated) (see **FIG. 6A( i )** to **FIG. 6A(iii)).**

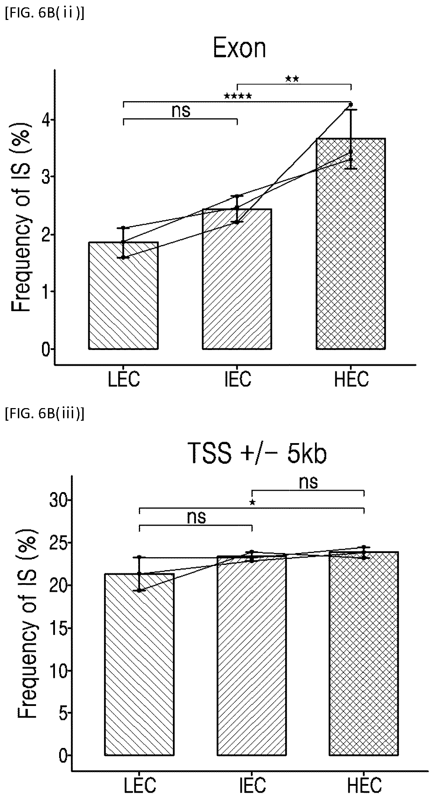

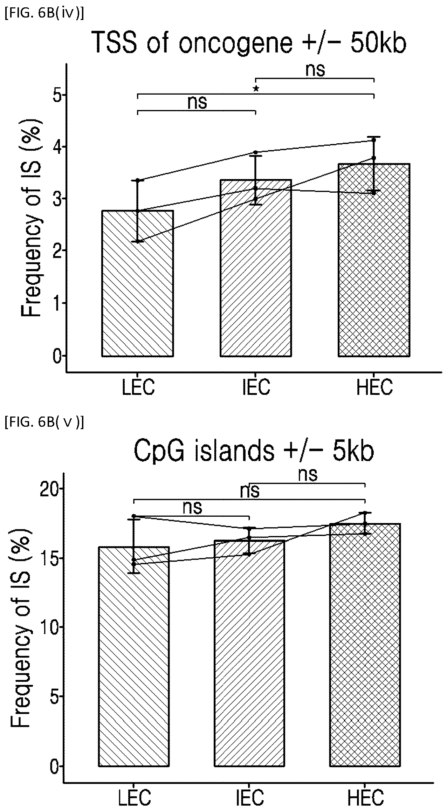

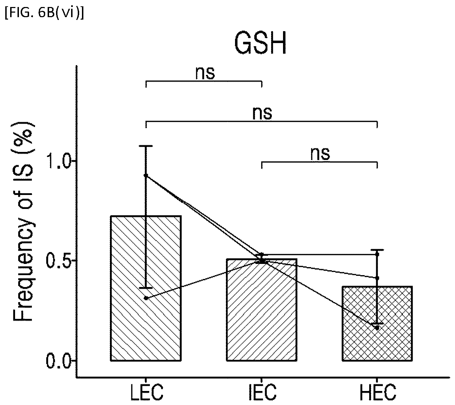

According to the result of analyzing the integration site ratios in the functionally important genomic regions depending on the clone abundance, it was found that the integration ratios vary depending on the clone abundance, as can be seen from **FIG. 6B(** i ) to **FIG. 6B(vi).**

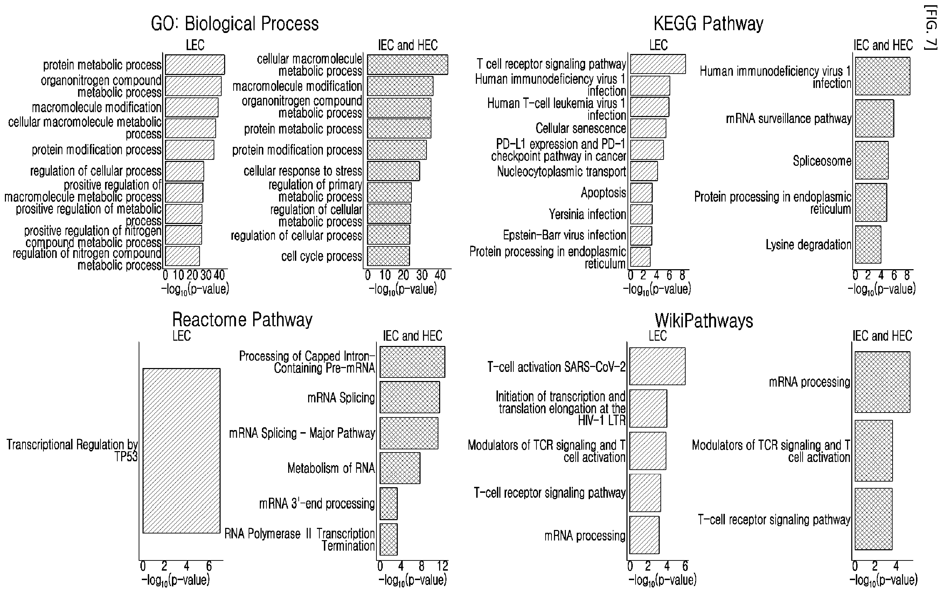

Meanwhile, according to the result of pathway enrichment analysis depending on the clone abundance in genes around an integration site, it was found that they are enriched in a conventionally known cellular metabolic pathway or a T cell-related pathway regardless of the clone abundance, as can be seen from **FIG. 7****.**

### D. Analysis of Integration Site in Vivo

The CAR-T cell line (cart006) prepared above was injected into mice. All tests were conducted with approval from the Institutional Animal Care and Use Committee of Seoul National University (SNUH-IACUC, 20-0177).

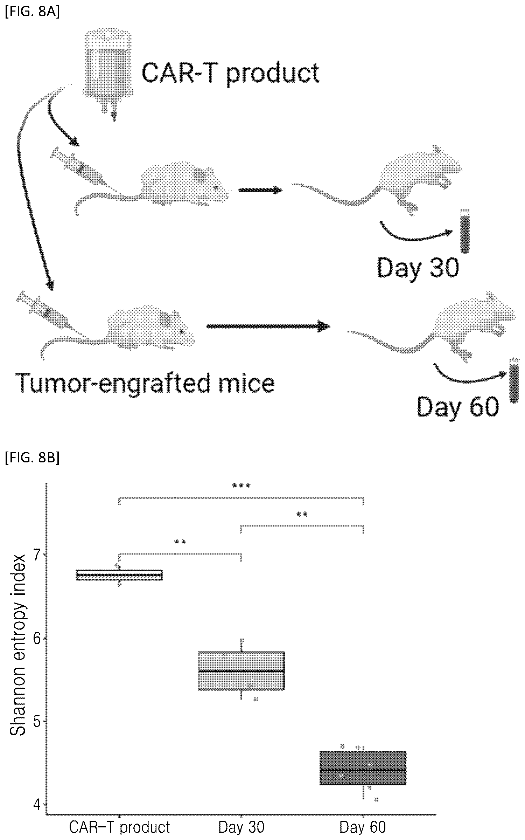

Specifically, 7-week-old immunodeficient NOD.Cg-Prkdcscid Il2rgtm1Wjl/SzJ (NSG) mice (10 mice in total) were intravenously through the tail vein with Luc-NALM-6 cells in an amount of 1.0 × 10⁵ per mouse. At 3 days after tumor cell inoculation, CD19 CAR-T cells suspended in saline solution were injected in an amount of 4.0 × 10⁶ per mouse, and the same volume of saline solution was administered to a control group. DNAs were extracted from the blood of CAR-T cells before injection, 4 mice at 30 days after injection (Day 30) and the remaining 6 mice at 60 days after injection (Day 60), and DIStinct-seq was performed (see **FIG. 8A****).**

First, the clone type and abundance were analyzed depending on the integration sites and the number of DNA fragments, and a difference in Shannon entropy index, an indicator of clonal diversity, between samples was measured. As can be seen from **FIG. 8B****,** the clonal diversity decreased in the Day 30 sample and further decreased in the Day 60 sample, compared to the CAR-T cells before injection.

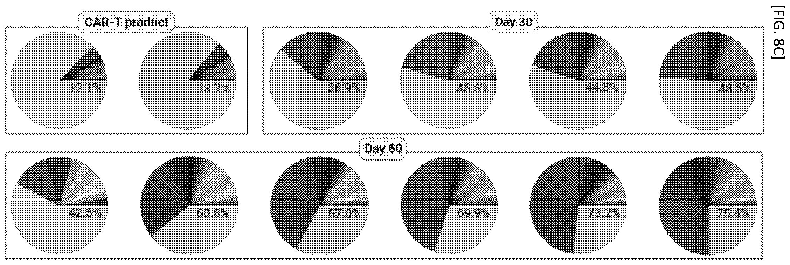

This tendency was also seen from the proportion of clones in the top 1 percent of clone abundance in all of clones of each sample (see **FIG. 8C****).** Particularly, although the number of types of clones decreased as the time increased to Day 60, certain clones did not proliferate monoclonally or oligoclonally, but polyclonal proliferation was observed without a dominant clone.

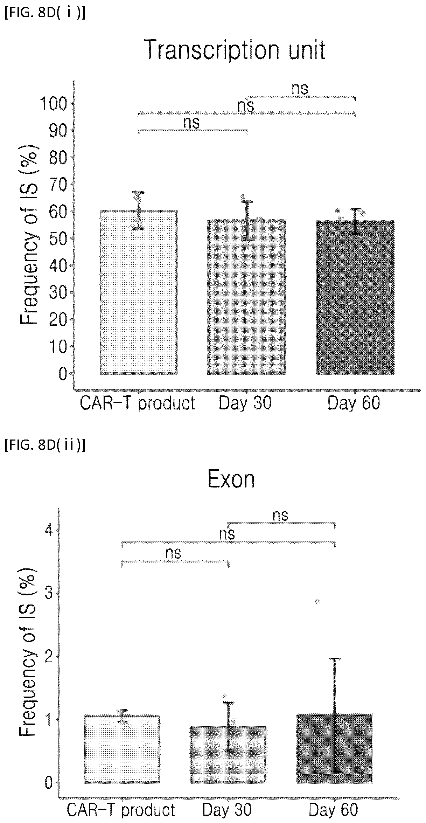

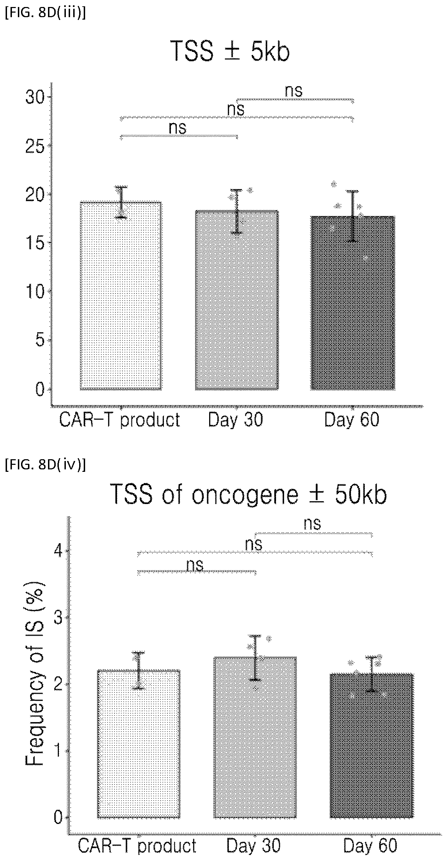

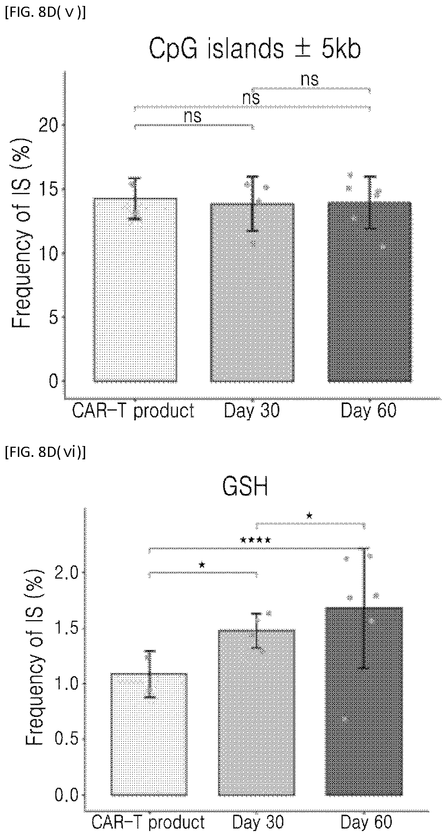

Also, according to the result of checking the integration ratios in the functional genomic regions, the insertion ratio in each genomic region was different depending on the time before and after injection, as can be seen from **FIG. 8D( i** ) to **FIG. 8D(vi).** Particularly, there was a statistically significant difference in genomic safe harbor (GSH) sites. This means that clonal persistence can be affected by the integration sites.

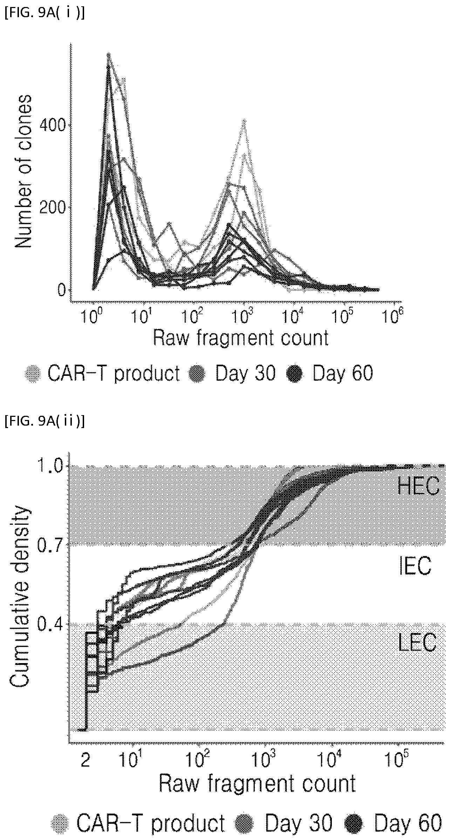

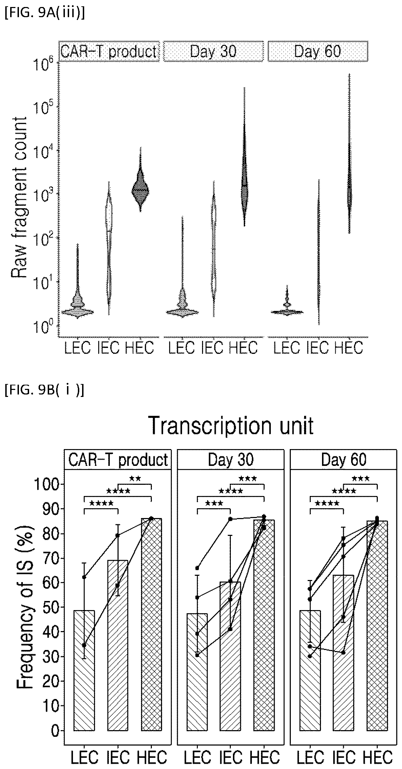

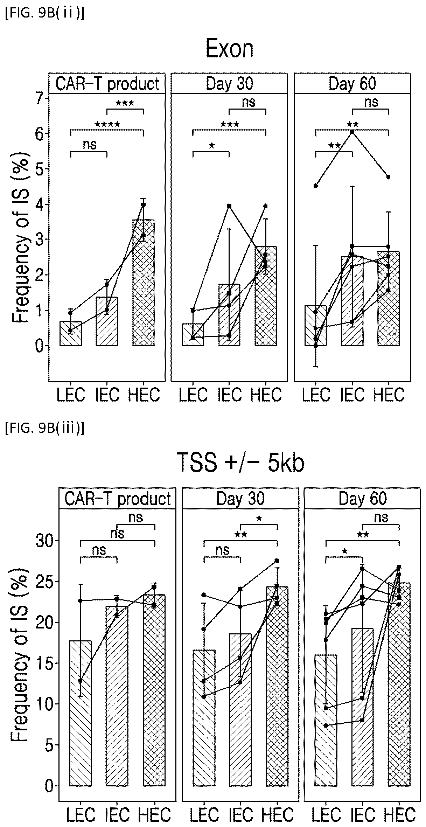

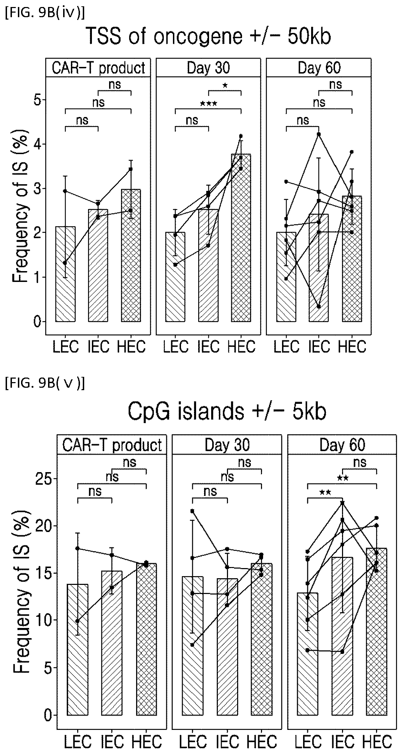

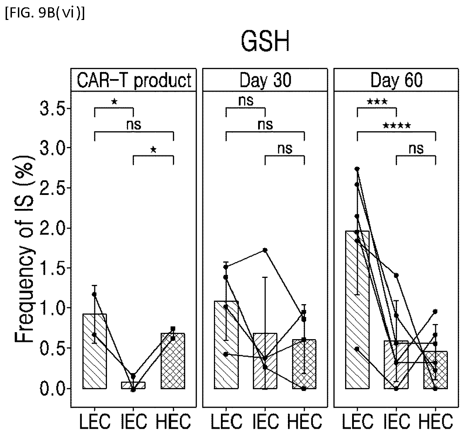

Further, when the clones were classified into LEC, IEC, and HEC depending on the number of DNA fragments with the same integration sites (clone abundance can be estimated) (see **FIG. 9A( i** ) to **FIG. 9A(iii)),** the integration ratios at some integration sites, such as transcription units, vary depending on the extent of clonal expansion. This means that the clonal expansion can also be affected by the integration sites.

## Claims

1. A method of detecting an integration site of a vector in a genome, comprising the following processes:
a process of tagmentation using a bead-linked transposome;
a process of preparing a library through gene amplification;
a process of library pooling and sequencing; and
a process of determining an integration site in a genome through bioinformatics analysis.

2. The method of detecting an integration site of a vector in a genome of Claim 1,
wherein in the process of tagmentation, fragmentation of DNA and adapter tagging are performed simultaneously.

3. The method of detecting an integration site of a vector in a genome of Claim 1,
wherein the process of preparing a library is performed by the following processes:
a 1st round polymerase chain reaction (PCR); and
a 2nd round PCR.

4. The method of detecting an integration site of a vector in a genome of Claim 3,
wherein the 1st PCR is performed in 30 cycles.

5. The method of detecting an integration site of a vector in a genome of Claim 3,
wherein the 2nd PCR is performed by a nested-PCR.

6. The method of detecting an integration site of a vector in a genome of Claim 3,
wherein elongation of the 1st PCR and the 2nd PCR is performed for 2 minutes.

7. The method of detecting an integration site of a vector in a genome of Claim 1,
wherein the process of determining an integration site in a genome is performed by the following processes:
a process of extracting a chimeric read;
a process of deleting a 3' LTR-specific sequence;
a process of creating a host/vector-fused genome;
a process of aligning the read on the host/vector-fused genome;
a process of removing a PCR duplicate;
a process of filtering the read; and
a process of determining the integration site.

8. The method of detecting an integration site of a vector in a genome of Claim 7,
wherein the process of determining an integration site in a genome uses a result value obtained by using at least one tool selected from the group consisting of Seqkit, Cutadapt, BWA, Picard, Samtools, and In-house Python script.

9. The method of detecting an integration site of a vector in a genome of Claim 1,
wherein the vector is a viral vector.

10. The method of detecting an integration site of a vector in a genome of Claim 9,
wherein the virus is selected from the group consisting of a lentivirus and a retrovirus.

11. The method of detecting an integration site of a vector in a genome of Claim 1,
wherein the method enables quantitative analysis of the integration site.

12. A method of quantifying clones with vectors integrated into genomes, comprising the following processes:
a process of tagmentation using a bead-linked transposome;
a process of preparing a library through gene amplification;
a process of library pooling and sequencing; and
a process of determining an integration site in a genome through bioinformatics analysis.
